(19) **Europäisches Patentamt / European Patent Office / Office européen des brevets**

(11) **EP 1 844 747 B1**

(12) **EUROPEAN PATENT SPECIFICATION**

(45) Date of publication and mention
of the grant of the patent:
**29.10.2014  Bulletin 2014/44**

(51) Int Cl.:
*B32B 29/00* (2006.01)     *B32B 5/02* (2006.01)
*B32B 5/26* (2006.01)     *B32B 7/02* (2006.01)
*A61F 13/02* (2006.01)     *C09J 7/02* (2006.01)

(21) Application number: **07007347.3**

(22) Date of filing: **10.04.2007**

(54) **Pressure-sensitive adhesive tape or sheet, and process for producing pressure-sensitive adhesive tape or sheet**

Haftklebeband oder -folie und Verfahren zur Herstellung des Haftklebebands oder der Haftklebefolie

Bande ou feuille adhésive sensible à la pression, et son procede de production

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **10.04.2006  JP 2006107114**
**20.06.2006  JP 2006170163**
**11.08.2006  JP 2006220499**

(43) Date of publication of application:
**17.10.2007  Bulletin 2007/42**

(73) Proprietor: **NITTO DENKO CORPORATION**
**Osaka (JP)**

(72) Inventors:
• **Funakoshi, Yoshio**
**Ibaraki-shi**
**Osaka (JP)**

• **Furumori, Kenji**
**Ibaraki-shi**
**Osaka (JP)**
• **Kasahara, Tsuyoshi**
**Ibaraki-shi**
**Osaka (JP)**
• **Ueda, Yuko**
**Ibaraki-shi**
**Osaka (JP)**
• **Hatanaka, Hiroshi**
**Ibaraki-shi**
**Osaka (JP)**

(74) Representative: **Grünecker, Kinkeldey,**
**Stockmair & Schwanhäusser**
**Leopoldstrasse 4**
**80802 München (DE)**

(56) References cited:
**EP-A1- 1 072 664       JP-A- 4 272 984**
**US-A1- 2003 040 691       US-B1- 6 506 489**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention relates to a pressure-sensitive adhesive tape or sheet, and more specifically relates to a pressure-sensitive adhesive tape or sheet useful for outer use in the medical and sanitary fields.

The invention also relates to a pressure-sensitive adhesive tape or sheet for medical use using substrates having a high void ratio and gentle to the skin, and a process for producing the same, and further relates to a pressure-sensitive adhesive tape or sheet capable of reducing manufacturing costs by direct application of an emulsion adhesive and excellent in feeling in use, and a process for producing the same.

BACKGROUND OF THE INVENTION

As pressure-sensitive adhesive tapes or sheets for medical use, for example, pressure-sensitive adhesive tapes including a support and a pressure-sensitive adhesive layer disposed on one side of the support are known, and these pressure-sensitive adhesive tapes are generally used by applying the pressure-sensitive adhesive layer to the skin. When a pressure-sensitive adhesive tape including a support having a high void ratio is used, the touch is good and a feeling of displeasure during the time of application is reduced, but when a pressure-sensitive adhesive layer is laminated on a support having a high void ratio, the adhesive excessively invades into the spaces of the support to reduce the adhesive strength, so that there arises a problem that the function as the pressure-sensitive adhesive tape or sheet is impaired. In consequence of that, when the support is subjected to a filling treatment for preventing invasion of adhesive, the void ratio lowers and the touch is deteriorated, and additionally, there are cases where stuffiness and eruption of skin due to physical stimulation by the edge part of the support occur.

For solving these problems, a method employing a nonwoven fabric as a support is proposed (Japanese Patent No. 3556082), but nonwoven fabrics have generally such a problem of incapable of sufficiently restraining the reduction of a void ratio, although they can easily retain a filling agent. Therefore, the problems of stuffiness and eruption of skin have not been solved yet. Accordingly, the development of a pressure-sensitive adhesive tape or sheet causing stuffiness and eruption of skin with difficulty and good to the touch is desired.

Further, a pressure-sensitive adhesive tape or sheet for medical use to be used by sticking on the skin is generally used by sticking on the skin to be applied via a pressure-sensitive adhesive layer. When a pressure-sensitive adhesive tape or sheet for medical use using a substrate having a high void ratio is used at this time, the touch is good and a feeling of displeasure during the time of application is reduced. However, when a substrate having a high void ratio is used, it is.usual to laminate an adhesive by transfer for preventing the adhesive solution from leaking to the rear face of the substrate through voids. In the case of transfer, for laminating an adhesive on a substrate, an operation to laminate the adhesive in advance on a sheet such as peeling paper, and stick the sheet on which the adhesive is laminated with the substrate to transfer the adhesive is necessary. However, according to the type of a pressure-sensitive adhesive tape or sheet for medical use, there is a case where a sheet such as peeling paper is unnecessary after the adhesive has been transferred to the substrate, and the sheet such as peeling paper is scrapped, so that manufacturing expenses pile up.

Considering the reduction of manufacturing costs and environmental aspect, lamination of an adhesive by direct application including applying an adhesive directly to a substrate is desired. However, when an adhesive is laminated on a substrate having a high void ratio by direct application, the adhesive invades into the spaces of the substrate, so that there arises a problem that the function as the pressure-sensitive adhesive sheet is lost. In addition, leaking of an adhesive to the rear face of a substrate is possible. When the adhesive solution leaks to the rear face of the substrate, the production for a long period of time is difficult, so that the yield at production conspicuously lowers.

When a substrate is subjected to a filling treatment for the purpose of prevention of invasion of an adhesive solution into the spaces of a substrate and leaking to the rear face of the substrate, the void ratio lowers and the touch worsens and in addition stuffiness and eruption of skin due to physical stimulation by the edge part of the substrate may occur.

In connection with a support for adhesive (a substrate), JP-A-10-67652 (the term "JP-A" as used herein refers to an "unexamined published Japanese patent application") discloses a method capable of direct application to the film face by combining a nonwoven fabric and a film to directly apply an adhesive solution. Considering the production stability, the method is useful, since the invasion of the adhesion solution and leaking to the rear face do not occur, but when the film is stuck on the skin for a long period of time, stuffiness is generated if the film does not have air permeability.

Accordingly, a pressure-sensitive adhesive tape or sheet for medical use difficult to be stuffy and good to the touch, and reduced in manufacturing costs is desired.

Further, a process for producing a pressure-sensitive adhesive tape or sheet for medical use difficult to be stuffy and good to the touch that is free from leakage of an adhesive solution to the rear face of a substrate at the time of direct application, and low in manufacturing costs is desired.

A relevant document in that respect is US 2003/0040691, disclosing a self-adhesive compressive elastic bandage which consists of multiple layers, and which is absorbent and breathable. The elastic bandage comprises a nonelastic absorbent nonwoven web, a nonelastic breathable nonwoven web, and a meltspun elastomeric material disposed between the two webs.

Patent document 1 : Japanese Patent No. 3556082
Patent document 2 : JP-A-10-67652
Patent document 3 : US 2003/0040691 A1

SUMMARY OF THE INVENTION

The invention has been done in view of such actual circumstances, and an object of the invention is to provide a pressure-sensitive adhesive tape or sheet difficult to cause stuffiness and eruption of skin and good to the touch.

As a result of intensive studies to solve the above problems, the present inventors have found that, by using a laminate including a combination of substrates different in properties as a support and arranging that an outermost layer is composed of a substrate having specific properties, an adhesive can be prevented from penetrating into the spaces of a support in the case of subjecting the support to a filling treatment and the void ratio can be inhibited from lowering, thus the above object can be achieved and the invention has been accomplished.

A pressure-sensitive adhesive tape or sheet according to the present invention is defined in claim 1.

Further advantageous embodiments are defined in claims 2 to 5.

BRIEF DESCRIPTION OF THE DRAWINGS

Fig. 1 is a drawing showing the cross section of a pressure-sensitive adhesive tape or sheet of one example in the invention.

Fig. 2 is a drawing showing the cross section of a pressure-sensitive adhesive tape or sheet of another example in the invention,

Fig. 3 is a drawing showing a substrate including three laminated layer (Examples 6 to 8, Comparative Examples 9 and 10).

Fig. 4 is a drawing showing a substrate including two laminated layer (Example 9).

Description of Reference Numerals

1: Support
2: Pressure-sensitive adhesive layer
3: First substrate
4: Second substrate
5: Outermost layer
10: Pressure-sensitive adhesive tape or sheet
20: Pressure-sensitive adhesive tape or sheet
31: Spunbond layer
32: Melt blow layer
33: Pressure-sensitive adhesive layer
34: Filling treatment and rear face treatment layer

DETAILED DESCRIPTION OF THE INVENTION

Namely, the invention relates to the following (1) to (5).

(1) A pressure-sensitive adhesive tape or sheet, is defined in claim 1.

(2) The pressure-sensitive adhesive tape or sheet according to (1), wherein the first substrate and second substrate are independently constituted of polyolefin, polyester, or polyamide.

(3) The pressure-sensitive adhesive tape or sheet according to (I), wherein the filling agent is in a form of emulsion.

(4) The pressure-sensitive adhesive tape or sheet according to (1), wherein the surface of the outermost layer on the support side is subjected to a release treatment.

(5) The pressure-sensitive adhesive tape or sheet according to (1), which is wound in a roll form.

In the invention, by using a laminate including a combination of first and second substrates having different properties as the support, and arranging that an outermost layer is composed of the second substrate, penetration of an adhesive into the spaces of the substrate can be prevented in a filling treatment of the support, and further the reduction of a void ratio can be prevented, so that a pressure-sensitive adhesive tape or sheet causing stuffiness and eruption of skin with difficulty and having a good touch can be provided. Accordingly, the pressure-sensitive adhesive tape or sheet in the invention is especially useful for medical use such as a sticking plaster and a dressing member.

The first embodiment of the invention that is one of the preferred embodiments will be described in detail below with reference to suitable examples. In the explanation of the figures, the same mark is affixed to the same element and duplicating explanation is omitted. Further, the dimensional ratios in the figures do not necessarily coincide with those in explanation for the convenience of illustration.

First Example:

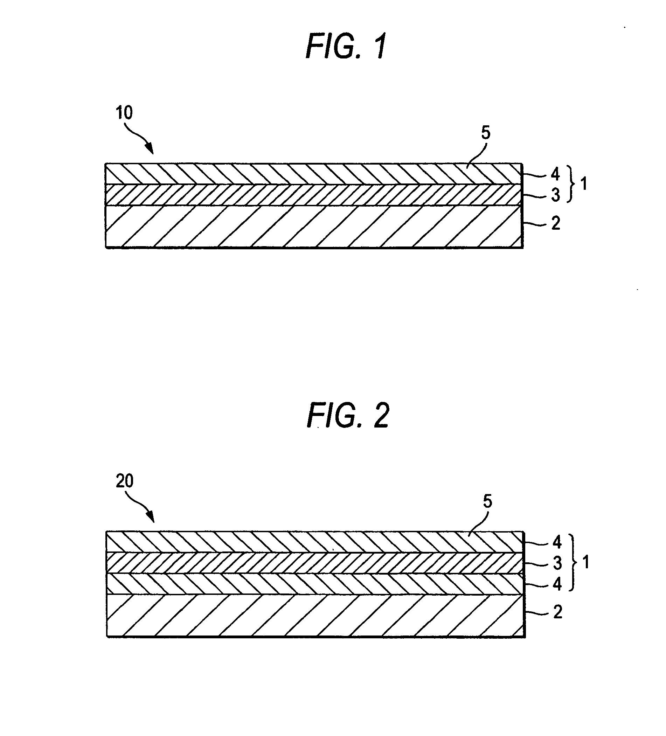

Fig. 1 is a drawing showing the cross section of a pressure-sensitive adhesive tape or sheet (hereinafter referred to as merely "a pressure-sensitive adhesive tape") of one example in the invention. Pressure-sensitive adhesive tape 10 according to the invention comprises a support 1 and a pressure-sensitive adhesive layer 2 disposed on one side of support 1, and the support 1 is treated with a filling agent. The support 1 is composed of a laminate of two-layer structure including a first substrate 3 and a second substrate 4, and the pressure-sensitive adhesive layer 2 is disposed on the first substrate 3 so that the second substrate 4 is arranged as an outermost layer 5 on the support side.

The first substrate includes a nonwoven fabric manufactured by a melt blowing method. By adopting a melt blow nonwoven fabric, stuffiness can be prevented due to the good air permeability, and the filling treatment becomes easy due to the high density of the melt blow nonwoven fabric. The kinds of materials of the first substrate are not particularly restricted so long as the materials are thermoplastic resins, and examples thereof include polyolefin (such as polyethylene and polypropylene), polyester, polyamide, polyacryl, and polyurethane, and polyolefin, polyester and polyamide are especially preferred.

The diameter of the fiber of the melt blow nonwoven fabric is not particularly restricted and a superfine fiber of 10 $\mu$m or less is generally used, and it is more preferred to use a fiber having a diameter of from 0.05 to 5 $\mu$m.

The thickness of the first substrate is 50 $\mu$m or less. When the thickness is more than 50 $\mu$m, the entire support becomes hard by the filling treatment, and the touch tends to be lowered. For the sufficient filling treatment, the thickness of the first substrate is preferably 5 $\mu$m or more.

The second substrate includes a nonwoven fabric manufactured by a spunbond method. By adopting a spunbond nonwoven fabric, the good touch can be obtained due to the high void ratio. The materials of the second substrate are not especially restricted so long as they are thermoplastic resins, and similarly to the first substrate, examples thereof include polyolefin (such as polyethylene and polypropylene), polyester, polyamide, polyacryl, and polyurethane, and polyolefin, polyester and polyamide are especially preferred. The material of the second substrate and the material of the first substrate may be the same or different.

The bulk density of the second substrate is from 0.05 to 0.3 g/cm$^3$, preferably from 0.08 to 0.28 g/cm$^3$, and more preferably from 0.1 to 0.25 g/cm$^3$. When the bulk density is more than 0.3 g/cm$^3$, the support becomes hard and the touch tends to be lowered, while when it is less than 0.05 g/cm$^3$, the substrate becomes too soft and difficult to handle. "Bulk density" used in the present specification means a value computed according to the following expression.

$$\text{Bulk density (g/cm}^3\text{)} = \text{the weight of the second substrate (g/cm}^2\text{)/the thickness of the second substrate (cm)}$$

The thickness of the second substrate is preferably 150 $\mu$m or less, and more preferably 100 $\mu$m or less. When the thickness is more than 150 $\mu$m, the substrate has a tendency to be difficult to handle in manufacturing a support and a pressure-sensitive adhesive tape or sheet, while when the thickness is less than 20 $\mu$m, the substrate is liable to be difficult to handle and at the same time feeling tends to be lowered. Accordingly, the thickness of the second substrate is preferably 20 $\mu$m or more.

The diameter of the fiber of a spunbond nonwoven fabric is not especially restricted, and it is generally 10 $\mu$m or more, and more preferably from 10 to 25 $\mu$m.

A support includes a first substrate and a second substrate laminated thereon, and ordinary methods can be used for the junction of both substrates, e.g., heat fusion and adhesion with an adhesive, can be used, and not especially restricted.

The support is treated with a filling agent, and the application amount of a filling agent is from 3 to 60 g/m$^2$, preferably from 5 to 50 g/m$^2$, and more preferably from 8 to 30 g/m$^2$. When the application amount is less than 3 g/m$^2$, sufficient filling treatment cannot be done, and in the case of forming a pressure-sensitive adhesive layer by directly applying an adhesive solution on the support face followed by drying, the adhesive easily soaks into the support, and there are cases where strike through of an adhesive to the rear face of the support occurs. When the application amount is more than 60 g/m$^2$, the entire support becomes hard and the touch tends to be deteriorated. Incidentally, when the filling agent is applied on the support, the filling agent tends to be present mainly on the first substrate that is manufactured by a melt blowing method and is close, so that the adhesive can be prevented from leaking out without impairing air permeability and feeling of the second substrate.

The air permeability of the support after the filling treatment is preferably from 0.1 to 100 sec/100 cc, more preferably from 0.1 to 70 sec/100 cc, and still more preferably from 0.1 to 30 sec/100 cc. When the air permeability is less than 0.1 sec/100 cc, the filling treatment is insufficient, and the adhesive easily soaks into the support similarly to the above. When it is more than 100 sec/100 cc, the support becomes hard and the touch tends to be lowered. Here, "air permeability" in the present specification means a value of an hour required for 100 cc of air to pass through a support after the filling treatment measured by Gurley densometer according to JIS L 1096.

A pressure-sensitive adhesive tape can be manufactured by, for example, forming a pressure-sensitive adhesive layer by applying an emulsion type adhesive on a support (on the side of a first substrate) and subsequent drying; or by applying an emulsion type adhesive on a substrate such as peeling paper and subsequent drying, followed by sticking the manufactured pressure-sensitive adhesive layer on the support (on the side of the first substrate). The shape of the pressure-sensitive adhesive tape may be not only plane (sheet-like) but also in a roll state, and the pressure-sensitive adhesive tape in a roll state can be manufactured by, for example, after forming a pressure-sensitive adhesive layer on a support (on the side of the first substrate), laying the surface of the pressure-sensitive adhesive layer on the support (on the side of the second substrate), and winding up in a roll state.

An emulsion type adhesive can be applied on a support with any of ordinarily used coaters, such as a gravure roll coater, a reverse roll coater, a kiss roll coater, a dip roll coater, a bar coater, a knife coater, and a spray coater. In this case, the emulsion type adhesive is applied so that the thickness of the pressure-sensitive adhesive layer after drying becomes generally from about 5 to 100 $\mu$m, preferably from about 10 to 60 $\mu$m, and more preferably from about 15 to 40 $\mu$m.

The surface of the outermost layer on the support side of the pressure-sensitive adhesive tape, that is, the opposite surface to the surface of the support on which the pressure-sensitive adhesive layer is formed (the second substrate side), may be subjected to a release treatment with a back surface treatment agent.

Thus, according to the invention, the adhesive can be prevented from soaking into the support and void ratio can be inhibited from lowering in the case of subjecting the support to a filling treatment, by using a laminate having a two-layer structure including a combination of the first substrate and the second substrate different in properties, and arranging that the outermost layer on the support side is composed of the second substrate. Accordingly, problems of stuffiness and eruption of skin can be get rid of, and a pressure-sensitive adhesive tape having a good touch can be manufactured, which is useful for medical use such as a first aid sticking plaster, a lengthy wrapping type sticking plaster, a large sticking plaster with a pad, and a dressing member.

Second Example:

Fig. 2 is a drawing showing the cross section of an adhesive tape of another example in the invention. Pressure-sensitive adhesive tape 20 comprises a support 1 and a pressure-sensitive adhesive layer 2 disposed on one side of the support 1. The Support 1 is composed of a laminate including a first substrate 3 and second substrates 4 disposed on both sides of the first substrate 3, and the second substrate 4 is arranged as outermost layer 5 on the support side. The pressure-sensitive adhesive tape of the second example is different from the pressure-sensitive adhesive tape of the first example comprising the laminate of two-layer structure in the point that the support is composed of the laminate of three-layer structure. Two second substrates constituting the support may be the same or different. The constitutions of the first and second substrates and the pressure-sensitive adhesive layer are the same as described in the first example.

Since the pressure-sensitive adhesive tape in the second example is composed of a laminate of three-layer structure, soaking of an adhesive and the reduction of the void ratio of the support after a filling treatment is still surely prevented. By this constitution, excellent feeling in use can be realized and a useful pressure-sensitive adhesive tape for medical use can be provided.

The pressure-sensitive adhesive tape in the invention has been described in detail above with reference to the examples, but the invention is not restricted to the above examples. Various changes and modifications can be made in the invention without departing from scope thereof. For example, for the purpose of protection of a pressure-sensitive adhesive layer, a release liner may be laminated on the pressure-sensitive adhesive layer, and release liners known in the field of the technique can be used. In the above examples, pressure-sensitive adhesive tapes for medical use such as a sticking plaster and the like are described as the use of the pressure-sensitive adhesive tape, and it is also possible to make a skin absorption type formulation of the pressure-sensitive adhesive tape by putting drugs into the pressure-sensitive adhesive layer.

In the next place, the second embodiment of the invention that is one of the preferred embodiments will be described in detail below.

The pressure-sensitive adhesive tape or sheet in the second embodiment of the invention has a laminate of a plurality of substrates and a pressure-sensitive adhesive layer disposed on at least one side of the laminate, in which the laminated substrates have different bulk densities, the laminated substrates are subjected to a filling treatment, and the loop hardness of the pressure-sensitive adhesive tape or sheet is from 15 to 60 mN.

In the invention, an adhesive is laminated on at least one side of the laminate of a plurality of substrates by direct application.

The loop hardness of the pressure-sensitive adhesive tape or sheet in the invention is from 15 to 60 mN, preferably from 15 to 50 mN, and more preferably from 15 to 40 mN. When the loop hardness is harder than 60 mN, a feeling of displeasure during the time of application occurs, while when the hardness is less than 15 mN, the filling treatment is insufficient and an adhesive cannot be laminated by direct application.

In the invention, the hardness of a loop is measured according to JIS L 1069 by a loop compression method.

In the invention, at least two layers of substrates are laminated. When only one layer is used, a large amount of a filling agent is required in applying the later-described filling treatment, so that drawbacks arise such that the touch of the pressure-sensitive adhesive tape or sheet for medical use is deteriorated and stuffiness tends to occur. Further, if only the substrates having a high bulk density are used for the easiness of the filling treatment, the touch of the substrate opposite to the pressure-sensitive adhesive layer becomes deteriorated. The above-mentioned at least two substrates including are members different in bulk density, preferably nonwoven fabrics different in bulk density. Filling treatment can be performed easily with a substrate having a high bulk density, and a substrate having a low bulk density can provide a good touch.

As such substrates, at least one of the laminated substrates is a nonwoven fabric having a bulk density of 0.25 g/cm$^3$ or less, and another one is a nonwoven fabric having a bulk density of 0.30 g/cm$^3$ or more. Preferably, at least one substrate may be a nonwoven fabric having a bulk density of 0.23 g/cm$^3$ or less, and another one may be a nonwoven fabric having a bulk density of 0.37 g/cm$^3$ or more. When the bulk density is 0.30 g/cm$^3$ or more, although the filling treatment can be performed easily, there is a problem in the point of the touch. When the bulk density is 0.25 g/cm$^3$ or less, although the touch is excellent, it is difficult to perform a sufficient filling treatment.

In the invention, bulk density can be computed by measuring the weight and the thickness of a substrate of 1 cm$^2$, and according to the following expression.

$$\textbf{Bulk density (g/cm}^3\textbf{)} = \textbf{the weight of a substrate}$$

$$\textbf{(g/cm}^2\textbf{)/the thickness of the substrate (cm)}$$

In the invention, "at least two layers of substrates are laminated" means that it is sufficient that two or more layers are laminated,'but for making the hardness of the pressure-sensitive adhesive tape or sheet for medical use from 15 to 60 mN, from 2 to 4 layers are laminated, and preferably 2 or 3 layers are laminated. The laminating method of substrates is not especially restricted, and ordinary methods such as heat fusion and a method with an adhesive can be used. By these methods, a nonwoven fabric having a bulk density of 0.25 g/cm$^3$ or less and a nonwoven fabric having a bulk density of 0.30 g/cm$^3$ or more can be laminated, or nonwoven fabrics having a bulk density of 0.25 g/cm$^3$ or less can be laminated on both sides of a nonwoven fabric having a bulk density of 0.30 g/cm$^3$ or more.

In the invention, as the members constituting the substrates, materials having a high melting point can be used. As such materials, polyesters are preferably used. As polyesters, specifically those excellent in heat resistance such as polyethylene terephthalate (PET) and polybutylene terephthalate (PBT) are more preferably used.

In the invention, a nonwoven fabric having a bulk density of 0.30 g/cm$^3$ or more can be manufactured by a melt blowing method. Further, a nonwoven fabric having a bulk density of 0.25 g/cm$^3$ or less can be manufactured by ordinary manufacturing methods of nonwoven fabrics. For example, as dry methods, adhesive type methods such as an immersion method, a print method, a spray method, a powder method and a thermal bond method, and mechanical bond type methods such as a felt method, a stitching method and a needle punch method, water jet slip type methods such as span lace method, spinning type methods such as a spunbond method, a network method, a melt blowing method and a film method are exemplified, and as wet methods, water jet slip type methods such as a span lace method, spinning type methods such as a spunbond method and a flash method, paper-making methods such as a heat fusion fiber method, a thermocompression method, and an adhesive method can be used.

In the invention, filling treatment is performed by applying from 10 to 50 $g/m^2$ of a filling agent on a substrate. When the application amount is less than 10 $g/m^2$, strike through of an adhesive cannot be prevented. On the other hand, when the application amount is more than 50 $g/m^2$, the substrate has a rough touch and easily becomes stuffy, and there may be cases where eruption of skin due to physical stimulation by the edge part of the substrate occurs. Accordingly, the application amount is preferably from 10 to 35 $g/m^2$, and more preferably from 10 to 20 $g/m^2$.

When a filling treatment is not performed, there are cases where an adhesive penetrates into a substrate in direct lamination of an emulsion adhesive on a substrate by direct application, or the function as the pressure-sensitive adhesive tape or sheet for medical use is lost by strike through of an emulsion adhesive and the like.

In the invention, the application amount of a filling agent can be computed by measuring the weight of 1 $m^2$ of the substrate applied with the filling agent and according to the following expression.

$$\text{Application amount of a filling agent } (g/m^2) = \text{the weight of the substrate applied with the filling agent } (g/m^2) - \text{the weight of the substrate } (g/m^2)$$

The pressure-sensitive adhesive tape or sheet in the invention has a pressure-sensitive adhesive layer at least on one side of the laminated substrates. The pressure-sensitive adhesive layer is preferably laminated on the side of the substrate having been subjected to a filling treatment by direct application. Since the substrate is subjected to a filling treatment, penetration and strike through of an adhesive can be prevented.

The form of the pressure-sensitive adhesive tape or sheet is not especially restricted and, for example, a pressure-sensitive adhesive tape in a roll state without employing a release paper, such as an adhesive member taking the roll form, is preferred. This is because a pressure-sensitive adhesive tape or sheet in a roll state does not require a release paper, so that the reducing effect of manufacturing costs by the laminating method of an adhesive by direct application is great. In the case of a pressure-sensitive adhesive tape or sheet in a roll state, the surface of the substrate on the side opposite to the pressure-sensitive adhesive layer is necessary to be subjected to back surface treatment.

Furthermore, the third embodiment of the invention that is the other preferred embodiment will be described in detail below.

The pressure-sensitive adhesive tape or sheet formed by the manufacturing method in the third embodiment of the invention comprises a laminate of a plurality of substrates and a pressure-sensitive adhesive layer disposed on at least one side thereof.

In the process not part of the invention, for effectively performing a filling treatment, it is necessary that at least two layers of substrates composed of paper and/or napkin should be laminated. When the substrate is composed of one layer, a large amount of a filling agent is required in applying the later-described filling treatment, so that drawbacks arise such that the touch of the pressure-sensitive adhesive tape or sheet for medical use becomes deteriorated and stuffiness tends to occur. Further, if only the substrates having a high bulk density are used for the easiness of the filling treatment, the touch of the substrate opposite to the pressure-sensitive adhesive layer becomes deteriorated. The above substrates including at least two layers are nonwoven fabrics different in bulk density. Filling treatment can be performed easily with a substrate having a high bulk density, and a substrate having a low bulk density can provide a good touch.

In the invention, substrates including nonwoven fabrics are used. For the members constituting these substrates materials having a high melting point are used. As such materials, polyesters are preferably used. As polyesters, specifically those excellent in heat resistance such as polyethylene terephthalate (PET) and polybutylena terephthalate (PBT) are more preferably used.

The loop hardness of the pressure-sensitive adhesive tape or sheet prepared according to the process of the invention is preferably 60 mN or less, more preferably 50 mN or less, and still more preferably 40 mN or less. This is because, when the loop hardness is harder than 60 mN, a feeling of displeasure during the time of application occurs. On the other hand, it is essential in the process not part of the invention that an adhesive solution be applied on a substrate by a direct application method, and the hardness of a loop is necessary to be 15 mN or more for that sake.

When the hardness of a loop is 15 mN or less, an adhesive solution cannot be applied by direct application.

In the invention, the hardness of a loop is measured according to JIS L 1069 by a loop compression method.

In the invention, "at least two layers of substrates are laminated" means it is sufficient that two or more layers are laminated, but for making the hardness of the obtained pressure-sensitive adhesive tape or sheet for medical use from 15 to 60 mN, from 2 to 4 layers are laminated, and preferably 2 or 3 layers are laminated. The substrates to be laminated, which include paper and/or napkin are preferably different in bulk density.

In the invention, bulk density can be computed by measuring the weight and the thickness of a substrate of 1 $cm^2$, and according to the following expression.

$$\texttt{Bulk density (g/cm}^3\texttt{)} = \texttt{the weight of a substrate}$$

$$\texttt{(g/cm}^2\texttt{)/the thickness of the substrate (cm)}$$

As such substrates, at least one of the laminated substrates is a nonwoven fabric having a bulk density of 0.25 $g/cm^3$ or less, and another one is a nonwoven fabric having a bulk density of 0.30 $g/cm^3$ or more. More preferably, at least one substrate may be a nonwoven fabric having a bulk density of 0.23 $g/cm^3$ or less, and another one may be a nonwoven fabric having a bulk density of 0.37 $g/cm^3$ or more. When the bulk density is 0.30 $g/cm^3$ or more, although the filling treatment can be performed easily, there is a problem in the point of the touch. When the bulk density is 0.25 $g/cm^3$ or less, although the touch is excellent, it is difficult to perform a sufficient filling treatment.

In the invention, a nonwoven fabric having a bulk density of 0.30 $g/cm^3$ or more can be manufactured by a melt blowing method. Further, a nonwoven fabric having a bulk density of 0.25 $g/cm^3$ or less can be manufactured by ordinary manufacturing methods of nonwoven fabrics. For example, as dry methods, adhesive type methods such as an immersion method, a print method, a spray method, a powder method and a thermal bond method, and mechanical bond type methods such as a felt method, a stitching method and a needle punch method, water jet slip type methods such as span lace method, spinning type methods such as a spunbond method, a network method, a melt blowing method and a film method are exemplified, and as wet methods, water jet slip type methods such as a span lace method, spinning type methods such as a spunbond method and a flash method, paper-making methods such as a heat fusion fiber method, a thermocompression method, and an adhesive method can be used.

The laminating method of substrates is not especially restricted, and ordinarily used methods, e.g., heat fusion, lamination by a melt blowing method, and a method with an adhesive can be used. For example, a layer of a nonwoven fabric having a low bulk density is formed by a spunbond method, and a layer of a polyester nonwoven fabric having a high bulk density can be laminated thereon by spraying polyester by a melt blowing method. According to the necessity, a polyester nonwoven fabric layer manufactured by a spunbond method can further be laminated thereon.

In the process not part of the invention, filling treatment by applying a filling agent on a substrate constituted of nonwoven fabric is necessary. If a substrate is not subjected to a filling treatment, there' are cases where an adhesive solution penetrates into the substrate in direct application, of the adhesive solution, or the function as the pressure-sensitive adhesive tape or sheet for medical use is lost by leaking of an emulsion adhesive solution to the rear face and the like.

In the invention, the application amount of a filling agent can be computed by measuring the weight of 1 $m^2$ of the substrate applied with the filling agent and according to the following expression.

Application amount of a filling agent ($g/m^2$) = the weight of the substrate applied with the filling agent ($g/m^2$) - the weight of the substrate ($g/m^2$)

In the invention, filling treatment is performed by applying from 10 to 50 $g/m^2$ of a filling agent on a substrate according to the above computation. When the application amount is less than 10 $g/m^2$, strike through of an adhesive solution to the rear surface cannot be prevented. On the other hand, when the application amount is more than 50 $g/m^2$, the substrate prepared by the process of the invention has a rough touch and easily becomes stuffy, and there may be cases where eruption of skin due to physical stimulation by the edge part of the substrate occurs. Accordingly, the application amount is preferably from 10 to 35 $g/m^2$, and more preferably from 10 to 20 $g/m^2$.

Application of a filling agent can be performed at a speed of from 5 to 30 m/minute, and in view of productivity, from 15 to 25 m/minute is preferred.

Drying after the application of a filling agent can be performed at the temperature in the range of from 120 to 170°C. Since expediting treating process is advantageous to productivity, the temperature of 140°c or more is preferred, and considering heat history to the substrate, the range of from 140 to 160°C is preferred.

In the process not part of the invention, the substrate subjected to a filling treatment is applied with an adhesive solution by direct application method.

Application of an adhesive solution by direct application method according to the process of the invention can be performed by a comma reverse method.

The viscosity of the adhesive solution that can be used in the invention is 50 Pa·s or higher. Taking uniform application of the adhesive solution into consideration, the viscosity in the range of from 50 to 150 Pa·s is preferred, and it is more preferably 50 to 130 Pa·s. The adhesive solution can be applied in a thickness of from 30 to 80 $\mu$m.

Application of the adhesive solution can be performed at a speed of from 5 to 30 m/minute, and it is more preferably in the range of from 15 to 25 m/minute from the viewpoint of productivity.

Drying after the application of the adhesive solution can be performed at a temperature in the range of from 120 to 180°C. Since expediting treating process is advantageous to productivity, the temperature of from 160 to 180°C is preferred for obtaining sufficient drying.

The form of the pressure-sensitive adhesive tape or sheet prepared by the process of the invention is not especially restricted and, for example, a pressure-sensitive adhesive tape in a roll state without employing a release paper, such as an adhesive member taking the roll form, is preferred. This is because a pressure-sensitive adhesive tape or sheet in a roll state does not require a release paper, so that the reducing effect of manufacturing costs by the laminating method of an adhesive by direct application method is great. In the case of a pressure-sensitive adhesive tape or sheet in a roll state, the surface of the substrate on the side opposite to the pressure-sensitive adhesive layer is necessary to be subjected to back surface treatment.

In the invention, filling agents are vinyl acetate series resins, acrylic series resins, styrene-butadiene rubber (SBR) series, rosin series resins. Solvent type filling agents may be used, but in view of the environmental protection, aqueous filling agents in an emulsion state are preferred. As the methods of filling treatment, ordinary methods can be used, for example, a method including adjusting a filling agent to an appropriate concentration, and impregnating a substrate with the filling agent, followed by drying, and the like.

Adhesives (adhesive solutions) to form a pressure-sensitive adhesive layer are not especially restricted so long as they show adhesion after coating and drying, and have good adhesion strength and retention. From the environmental protection, emulsion type adhesives (adhesive solutions) are preferably used. As emulsion type adhesives, for example, aqueous dispersions of acrylic polymers and urethane-acrylic polymers, and aqueous dispersions of rubber polymers such as synthetic rubbers, e.g., styrene-butadiene copolymers, and natural rubbers are exemplified. Further, according to the necessity, adhesives may be blended with additives generally used in this technical field such as crosslinking agents, tackifier resins, fillers, oils, and pigments.

Incidentally, as the above-described back surface treatment agents, fluorine type release agents, long chain alkyl type release agents, silicone type release agents, or mixtures thereof can be used. As the method of release treatment, ordinarily methods used in this technical field can be used.

EXAMPLE

The invention will be described more specifically with reference to examples, but the invention is not restricted thereto, and various changes and modifications can be made in the invention without departing from the technical thought of the invention.

In the examples, "parts" means "weight parts" unless otherwise indicated.

The examples concerning the first embodiment of the invention are described below.

In the examples, the preparation of an adhesive and the manufacture of a pressure-sensitive adhesive layer were carried out according to the following methods.

Preparation of an emulsion adhesive

To a monomer mixture containing 95 weight parts of 2-ethylhexyl acrylate and 5 weight parts of acrylic acid were added 2 weight parts of sodium lauryl sulfate (an emulsifier), and 0.2 weight parts of potassium persulfate (a polymerization initiator), and the mixture was emulsion polymerized according to a conventional method to obtain an emulsion adhesive containing an acrylic polymer (the weight average molecular weight of the solvent-soluble polymer: 800,000) uniformly emulsion dispersed in water. The obtained adhesive had solids content concentration of 50wt%, and the content of gel (crosslinking content) of 46wt%.

Manufacture of a pressure-sensitive adhesive layer

The above-obtained emulsion adhesive was applied on a polyethylene terephthalate substrate having been subjected to release treatment, and dried at 120°C for 3 minutes to obtain a pressure-sensitive adhesive layer having a thickness of 40 $\mu$m.

EXAMPLE 1

After laminating nonwoven fabrics manufactured by a spunbond method and having a bulk density of 0.21 g/cm$^3$ (made of polyester, a thickness: 80 μm, a fiber diameter: 14 μm) on both sides of a nonwoven fabric manufactured by a melt blowing method (made of polyester, a thickness: 25 μm, a fiber diameter: 1.8 μm), 25 g/m$^2$ of a filling agent was applied for a filling treatment, thus a filling-treated support was obtained. Voncort R3360 (trade name, manufactured by Dainippon Ink and Chemicals Inc.) was used as the filling agent. The air permeability of the obtained support was 12 sec/100 cc. Subsequently, the above-obtained pressure-sensitive adhesive layer was transferred to the filling-treated support to obtain a pressure-sensitive adhesive sheet.

EXAMPLE 2

After laminating nonwoven fabrics manufactured by a spunbond method and having a bulk density of 0.12 g/cm$^3$ (made of polyester, a thickness: 45 μm, a fiber diameter: 14 μm) on both sides of a nonwoven fabric manufactured by a melt blowing method (made of polyester, a thickness: 25 μm, a fiber diameter: 1.8 μm), 30 g/m$^2$ of the same filling agent as used in Example 1 was coated, and a filling-treated support was obtained. The air permeability of the obtained support was 30 sec/100 cc. Subsequently, the above-obtained pressure-sensitive adhesive layer was transferred to the filling-treated support to obtain a pressure-sensitive adhesive sheet.

COMPARATIVE EXAMPLE 1

The same filling agent as used in Example 1 was coated on a nonwoven fabric manufactured by a spunbond method and having a bulk density of 0.21 g/cm$^3$ (made of polyester, a thickness: 120 μm, a fiber diameter: 13 μm), and a support including a single layer was obtained. The air permeability of the obtained support was 1 sec/100 cc. Subsequently, the above-obtained pressure-sensitive adhesive layer was transferred to the filling-treated support, but the adhesive penetrated into the support, so that a pressure-sensitive adhesive sheet could not be obtained.

COMPARATIVE EXAMPLE 2

Nonwoven fabrics manufactured by a spunbond method and having a bulk density of 0.21 g/cm$^3$ (made of polyester, a thickness: 45 μm, a fiber diameter: 13 μm) was laminated on both sides of a nonwoven fabric manufactured by a melt blowing method (made of polyester, a thickness: 25 μm, a fiber diameter: 1.8 μm) to obtain a support. The air permeability of the obtained support not subjected to filling treatment was 0.08 sec/100 cc. Subsequently, the above-obtained pressure-sensitive adhesive layer was transferred to the support, but the adhesive penetrated into the support, so that a pressure-sensitive adhesive sheet could not be obtained.

COMPARATIVE EXAMPLE 3

The same filling agent (30 g/m$^2$) as used in Example 1 was applied on a nonwoven fabric manufactured by a melt blowing method and having a bulk density of 0.12 g/cm$^3$ (made of polyester, a thickness : 70 μm, a fiber diameter: 1.8 μm) to obtain a filling-treated support. The air permeability of the obtained support was 30 sec/100 cc. Subsequently, the above-obtained pressure-sensitive adhesive layer was transferred to the filling-treated support to obtain a pressure-sensitive adhesive sheet.

Evaluation Tests

Air permeability:

In connection with the air permeability of the supports used in Examples 1 and 2 and Comparative Examples 1 to 3, the hour required for 100 ml of air to pass through each support was measured by Gurley densometer according to JIS L 1069. The results of measurement are shown in Table 1 below.

Bulk density:

The bulk density of each of the second substrates used in Examples 1 and 2 and Comparative Examples 1 to 3 was computed according to the following expression. The results obtained are shown in Table 1 below.

$$\text{Bulk density (g/cm}^3) = \text{the weight of the second substrate (g/cm}^2)/\text{the thickness of the second substrate (cm)}$$

Penetration of adhesive:

Pressure-sensitive adhesive sheets obtained in Examples 1 and 2 and Comparative Examples 1 to 3 were subjected to aging at 60°C for 3 days, and the sticking property of each adhesive sheet was evaluated according to the following criteria. The results obtained are shown in Table 1.

    A: Sticky and sticking on the skin is possible.
    B: Free of stickiness and sticking on the skin is impossible.

Touch:

Each outermost layer of the support of the pressure-sensitive adhesive sheets obtained in Examples 1 and 2 and Comparative Examples 1 to 3 was touched with a hand, and the touch was evaluated according to the following criteria. The results of evaluation are shown in Table 1.

    A: Good to the touch.
    B: The touch is a little inferior.
    C: Hard and rough to the touch.

**TABLE 1**

| Example No. | Material of Outermost Layer | Bulk Density of Second Substrate (g/cm³) | Coating Amount of Filling Agent (g/cm²) | Air Permeability of Support (sec/100ml) | Penetration of Adhesive | Touch |
|---|---|---|---|---|---|---|
| Example 1 | Spunbond Nonwoven Fabric | 0.21 | 25 | 12 | A | A |
| Example 2 | Spunbond Nonwoven Fabric | 0.12 | 30 | 30 | A | A |
| Comparative Example 1 | Spunbond Nonwoven Fabric | - | 25 | 1.0 | B | - |
| Comparative Example 2 | Spunbond Nonwoven Fabric | 0.17 | 0 | 0.08 | B | - |
| Comparative Example 3 | Melt blow Nonwoven Fabric | - | 30 | 30 | A | B |

It has been confirmed that the pressure-sensitive adhesive sheets obtained in Examples 1 and 2 do not cause strike through of the adhesive and the touch is good, since each of the supports has specific lamination structure and the outermost layer is composed of a spunbond nonwoven fabric.

Contrary to this, the pressure-sensitive adhesive sheet in Comparative Example 1 causes penetration of the adhesive, since the support does not have a specific lamination structure. The pressure-sensitive adhesive sheet in Comparative Example 2 causes penetration of the adhesive and cannot function as the pressure-sensitive adhesive sheet, since it is not subjected to a filling treatment, although the support has a specific lamination structure. Further, the pressure-sensitive adhesive sheet in Comparative Example 3 does not cause striking through of the adhesive, since the support is composed of a melt blow nonwoven fabric, but the touch is not good, since the support is arranged as the outermost layer.

Subsequently, the examples concerning the second embodiment of the invention are described below.

1. Process for producing a pressure-sensitive adhesive sheet

1) Preparation of a polymer for forming a pressure-sensitive adhesive layer

By emulsion polymerizing a monomer mixture containing 95 parts of 2-ethylhexyl acrylate and 5 parts of acrylic acid by using 2 parts of sodium lauryl sulfate (an emulsifier) and 0.2 parts of potassium persulfate (a polymerization initiator) according to a conventional method, an emulsion adhesive having a solid content concentration of 50wt%, in which an acrylic polymer containing 46wt% of gel content in particles (crosslinking content) and having a weight average molecular weight of the solvent-soluble polymer of 800,000 is uniformly emulsion dispersed in water, was obtained. The obtained emulsion adhesive was employed as a polymer for forming a pressure-sensitive adhesive layer.

2) Filling treatment

An acryl ester copolymer emulsion (Tg: -30°C) was used as the filling agent. A substrate was impregnated with the filling agent and dried at 120°C for 2 minutes.

3) Formation of a pressure-sensitive adhesive layer

Forming method of a pressure-sensitive adhesive layer by direct application:

The above-prepared polymer for forming a pressure-sensitive adhesive layer was applied on one side of the substrate in a coating thickness of 40 μm, followed by drying at 130°C for 3 minutes to obtain an adhesive sheet.

Forming method of a pressure-sensitive adhesive layer by transfer:

The above-prepared polymer for forming a pressure-sensitive adhesive layer was applied on a release paper in a coating thickness of 40 μm, followed by drying at 130°C for 3 minutes to thereby form a pressure-sensitive adhesive layer in advance. The adhesive on the release paper was transferred to a substrate to obtain a pressure-sensitive adhesive sheet.

2. Test method

Pressure-sensitive Aadhesive sheets for sticking on the skin obtained in each example and comparative example were tested as follows.

1) Application amount of the filling agent

The application amount of the filling agent was computed by measuring the weight of 1 $m^2$ of the substrate coated with the filling agent and according to the following expression.

$$\text{Application amount of the filling agent } (g/m^2) = \text{the weight of the substrate applied with the filling agent } (g/m^2) - \text{the weight of the substrate } (g/m^2)$$

2) Bulk density

Bulk density was computed by measuring the weight and the thickness of the substrate of 1 $cm^2$ and according

to the following expression.

$$\texttt{Bulk density (g/cm}^3\texttt{) = the weight of the substrate}$$

$$\texttt{(g/cm}^2\texttt{)/the thickness of the substrate (cm)}$$

3. Evaluating methods

Pressure-sensitive adhesive sheets for sticking on the skin obtained in each example and comparative example were evaluated as follows.

    1) Formation of a pressure-sensitive adhesive sheet
    2) Sticking through of the polymer for forming a pressure-sensitive adhesive layer

After forming a pressure-sensitive adhesive layer on one side of the substrate, whether the polymer for forming a pressure-sensitive adhesive layer soaked on the opposite side to the pressure-sensitive adhesive layer or not was functionally evaluated.

    A: Free from soaking
    B: Soaked and wet.
    C: Soaked and sticky.

3) Hardness of loop

The loop hardness was measured according to JIS L 1069 by a loop compression method.

4) Observation at the time of sticking

A pressure-sensitive adhesive sheet having a size of 25 mm x 50 mm was stuck on the forearm and stuffiness and eruption of skin were functionally observed.

    A: Free from stuffiness and free from eruption of skin.
    B: Accompanied by stuffiness and eruption of skin.

EXAMPLE 3

A substrate was prepared by laminating polyethylene terephthalate nonwoven fabrics having a bulk density of 0.23 $g/cm^3$ and manufactured by a spunbond method on both sides of a polyethylene terephthalate nonwoven fabric having a bulk density of 0.37 $g/cm^3$ and manufactured by a melt blowing method. Then the substrate was applied with 15 $g/m^2$ of the filling agent, and the polymer for forming a pressure-sensitive adhesive layer was directly applied on one side of the substrate in a thickness of 40 $\mu$m according to the forming method of a pressure-sensitive adhesive layer by direct application, thus a pressure-sensitive adhesive sheet having the hardness of loop of 26 mN was obtained. Strike through of the polymer for forming a pressure-sensitive adhesive layer was not confirmed at this time. There was also no problem on stuffiness when the pressure-sensitive adhesive sheet was stuck on the skin, and also stimulation to the skin was not confirmed.

EXAMPLE 4

A substrate was prepared by laminating polyethylene terephthalate nonwoven fabrics having a bulk density of 0.23 $g/cm^3$ and manufactured by a spunbond method on both sides of a polyethylene terephthalate nonwoven fabric having a bulk density of 0.37 $g/cm^3$ and manufactured by a melt blowing method. The substrate was applied with 25 $g/m^2$ of the filling agent, and the polymer for forming a pressure-sensitive adhesive layer was directly applied on one side of the substrate in a thickness of 40 $\mu$m according to the forming method of a pressure-sensitive adhesive layer by direct application, thus a pressure-sensitive adhesive sheet having the hardness of loop of 30 mN was obtained. Strike through of the polymer for forming a pressure-sensitive adhesive layer was not confirmed at this time. There was also no problem on stuffiness when the pressure-sensitive adhesive sheet was stuck on the skin, and also stimulation to the skin was not confirmed.

EXAMPLE 5

A substrate was prepared by laminating a polyethylene terephthalate nonwoven fabric having a bulk density of 0.16 g/cm$^3$ and manufactured by a spunbond method on one side of a polyethylene terephthalate nonwoven fabric having a bulk density of 0.30 g/cm$^3$ and manufactured by a melt blowing method. The substrate was applied with 40 g/m$^2$ of the filling agent, and the polymer for forming a pressure-sensitive adhesive layer was directly applied on the side of the polyethylene terephthalate nonwoven fabric having a bulk density of 0.30 g/cm$^3$ manufactured by a melt blowing method in a thickness of 40 $\mu$m according to the forming method of a pressure-sensitive adhesive layer by direct application, thus a pressure-sensitive adhesive sheet having the hardness of loop of 15 mN was obtained. Strike through of the polymer for forming a pressure-sensitive adhesive layer was not confirmed at this time. There was also no problem on stuffiness when the pressure-sensitive adhesive sheet was stuck on the skin, and also stimulation to the skin was not confirmed.

COMPARATIVE EXAMPLE 4

A substrate was prepared by laminating polyethylene terephthalate nonwoven fabrics having a bulk density of 0.23 g/cm$^3$ and manufactured by a spunbond method a on both sides of a polyethylene terephthalate nonwoven fabric having a bulk density of 0.37 g/cm$^3$ and manufactured by a melt blowing method. The substrate was applied with 30 g/m$^2$ of the filling agent, and the polymer for forming a pressure-sensitive adhesive layer was directly applied on one side of the substrate in a thickness of 40 $\mu$m according to the forming method of a pressure-sensitive adhesive layer by direct application, thus a pressuxe-sensitive adhesive sheet having the hardness of loop of 72 mN was obtained. Strike through of the polymer for forming a pressure-sensitive adhesive layer was not confirmed at this time. Stuffiness was confirmed when the pressure-sensitive adhesive sheet was stuck on the skin, and stimulation to the skin was confirmed at the edge part of the pressure-sensitive adhesive sheet.

COMPARATIVE EXAMPLE 5

A substrate was prepared by laminating polyethylene terephthalate nonwoven fabrics having a bulk density of 0.23 g/cm$^3$ and manufactured by a spunbond method on both sides of a polyethylene terephthalate nonwoven fabric having a bulk density of 0.37 g/cm$^3$ and manufactured by a melt blowing method. The substrate was applied with 10 g/m$^2$ of the filling agent, and the polymer for forming a pressure-sensitive adhesive layer was directly applied on one side of the substrate in a thickness of 40 $\mu$m according to the forming method of a pressure-sensitive adhesive layer by direct application to try to manufacture a pressure-sensitive adhesive sheet having the hardness of loop of 22 mN. However, strike through of the polymer for forming a pressure-sensitive adhesive layer was confirmed on this condition, and a pressure-sensitive adhesive sheet could not be obtained.

COMPARATIVE EXAMPLE 6

When the polymer for forming a pressure-sensitive adhesive layer was directly applied in a thickness of 40 $\mu$m on one side of a substrate including a polyethylene terephthalate nonwoven fabric having a bulk density of 0.37 g/cm$^3$ and manufactured by a melt blowing method having laminated on both sides thereof polyethylene terephthalate nonwoven fabrics having a bulk density of 0.23 g/cm$^3$ and manufactured by a spunbond method according to the forming method of a pressure-sensitive adhesive layer by direct application. Strike through of the polymer for forming a pressure-sensitive adhesive layer was confirmed, and a pressure-sensitive adhesive sheet could not be obtained.

COMPARATIVE EXAMPLE 7

A substrate was prepared by laminating a polyethylene terephthalate nonwoven fabric having a bulk density of 0.16 g/cm$^3$ and manufactured by a spunbond method on one side of a polyethylene terephthalate nonwoven fabric having a bulk density of 0.30 g/cm$^3$ and manufactured by a melt blowing method. The substrate was applied with 20 g/m$^2$ of the filling agent, and the polymer for forming a pressure-sensitive adhesive layer was directly applied on the side of the polyethylene terephthalate nonwoven fabric having a bulk density of 0.30 g/cm$^3$ and manufactured by a melt blowing method in a thickness of 40 $\mu$m according to the forming method of a pressure-sensitive adhesive layer by direct application. Strike through of the polymer for forming a pressure-sensitive adhesive layer was confirmed, and a pressure-sensitive adhesive sheet could not be obtained.

COMPARATIVE EXAMPLE 8

On the side of a polyethylene film of a substrate including a laminate of a polyethylene film having a thickness of 8 μm and a polyethylene terephthalate nonwoven fabric having a bulk density of 0.23 g/cm$^3$ and manufactured by a spunbond method was transferred the polymer for forming a pressure-sensitive adhesive layer formed in advance in a thickness of 40 μm according to the forming method of a pressure-sensitive adhesive layer by transfer to thereby obtain a pressure-sensitive adhesive sheet. Strike through of the polymer for forming a pressure-sensitive adhesive layer was not confirmed at this time. Stimulation to the skin was not confirmed when the pressure-sensitive adhesive sheet was stuck on the skin, but stuffiness occurred.

The pressure-sensitive adhesive sheets obtained in Examples 3 to 5 and Comparative Examples 4 to 8 are summarized in Table 2 below.

TABLE 2

| Example No. | Application Amount of Filling Agent (g/m$^2$) | Bulk Density (g/cm$^3$) | | Constitution of Substrate |
|---|---|---|---|---|
| | | MB Layer | SB Layer | |
| Example 3 | 15 | 0.37 | 0.23 | 3-Layer structure |
| Example 4 | 20 | 0.37 | 0.23 | 3-Layer structure |
| Example 5 | 40 | 0.3 | 0.16 | 2-Layer structure |
| Comparative Example 4 | 30 | 0.37 | 0.23 | 3-Layer structure |
| Comparative Example 5 | 10 | 0.37 | 0.23 | 3-Layer Structure |
| Comparative Example 6 | 0 | 0.37 | 0.23 | 3-Layer structure |
| Comparative Example 7 | 20 | 0.3 | 0.16 | 2-Layer structure |
| Comparative Example 8 | 0 | - | 0.23 | 2-Layer structure |
| MB layer: Nonwoven fabric layer formed by a melt blowing method SB layer: Nonwoven fabric layer formed by a spunbond method | | | | |

The results of evaluations of the pressure-sensitive adhesive sheets obtained in Examples 3 to 5 and Comparative Examples 4 to 8 are shown in Table 3 below.

TABLE 3

| Example No. | Whether Adhesive Sheet Could Be Formed Or Not | Degree of Strike Through of Adhesive | Hardness of Loop (mN) | Stuffiness | Eruption of Skin |
|---|---|---|---|---|---|
| Example 3 | YES | A | 26 | A | A |
| Example 4 | YES | A | 30 | A | A |
| Example 5 | YES | A | 15 | A | A |
| Comparative Example 4 | YES | A | 72 | B | B |
| Comparative Example 5 | NO | B | (22) | - | - |
| Comparative Example 6 | NO | - | - | - | - |
| Comparative Example 7 | NO | C | - | - | - |

(continued)

| Example No. | Whether Adhesive Sheet Could Be Formed Or Not | Degree of Strike Through of Adhesive | Hardness of Loop (mN) | Stuffiness | Eruption of Skin |
|---|---|---|---|---|---|
| Comparative Example 8 | YES | A | 13 | B | A |

Further, the examples concerning the third embodiment of the invention are described below.

1. Process for producing a pressure-sensitive adhesive sheet

1) Preparation of an emulsion adhesive solution

By emulsion polymerizing a monomer mixture containing 95 parts of 2-ethylhexyl acrylate and 5 parts of acrylic acid by using 2 parts of sodium lauryl sulfate (an emulsifier) and 0.2 parts of potassium persulfate (a polymerization initiator) according to a conventional method, an emulsion adhesive solution having a solid content concentration of 50wt%, in which an acrylic polymer containing a gel content in particles (crosslinking content) of 46wt% and having a weight average molecular weight of the solvent-soluble polymer of 800,000 is uniformly emulsion dispersed in water, was obtained.

2) Filling treatment

An acryl ester copolymer emulsion (Tg: -30°C) was used as the filling agent. A substrate was impregnated with the filling agent and dried at 120°C for 2 minutes.

3) Application of an adhesive solution by direct application

The prepared emulsion adhesive solution was applied by a comma reverse method on a substrate in a thickness of 40 μm, and dried at 130°C for 3 minutes to obtain a pressure-sensitive adhesive sheet.

2. Test methods

Pressure-sensitive adhesive sheets for sticking on the skin obtained in each example and comparative example were tested as follows.

1) Application amount of the filling agent

The application amount of the filling agent was computed by measuring the weight of 1 $m^2$ of the substrate applied with the filling agent and according to the following expression.

$$\text{Application amount of the filling agent } (g/m^2) =$$
$$\text{the weight of the substrate applied with the}$$
$$\text{filling agent } (g/m^2) - \text{the weight of the substrate}$$
$$(g/m^2)$$

2) Coefficient of viscosity of the adhesive solution

Coefficient of viscosity was measured with a BH type viscometer (manufactured by TOKI SANGYO CO., LTD.).

3. Evaluating methods

Pressure-sensitive adhesive sheets for medical use obtained in each example and comparative example were evaluated as follows.

1) Leaking to the rear face of the substrate

After coating the adhesive solution on the substrate and before performing drying process, whether the adhesive solution leaked to the rear face of the substrate or not was confirmed by feeling with fingers.

2) Possibility of adhesive solution coating

Whether the adhesive solution can be uniformly applied on the substrate or not was visually observed.

3) Hardness of loop

The loop hardness was measured according to JIS L 1069 by a loop compression method.

EXAMPLE 6

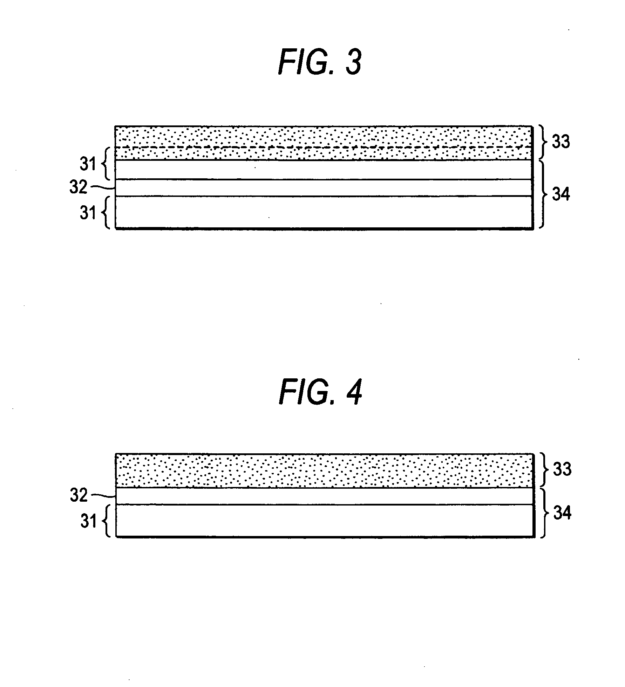

After applying 15 g/m$^2$ of the filling agent in the solution concentration of 23% on a substrate including a Polyethylene terephthalate nonwoven fabric having a bulk density of 0.37 g/cm$^3$ and manufactured by a melt blowing method having laminated on both sides thereof polyethylene terephthalate nonwoven fabrics having a bulk density of 0.23 g/cm$^3$ and manufactured by a spunbond method (Fig. 3), the emulsion adhesive solution having a viscosity of 70 Pa·s was applied in a thickness of 40 $\mu$m. As a result, leaking of the adhesive solution to the rear side of the substrate was not confirmed and coating by direct application method was possible. The hardness of loop of the pressure-sensitive adhesive sheet at this time was 26 mN.

EXAMPLE 7

After applying 20 g/m$^2$ of the filling agent in the solution concentration of 45% on the substrate manufactured in the same manner as in Example 6 (Fig. 3), the emulsion adhesive solution having a viscosity of 70 Pa·s was applied in a thickness of 40 $\mu$m. As a result, leaking of the adhesive solution to the rear side of the substrate was not confirmed and coating by direct application method was possible. The hardness of loop of the pressure-sensitive adhesive sheet at this time was 30 mN.

EXAMPLE 8

After applying 15 g/m$^2$ of the filling agent in the solution concentration of 23% on the substrate manufactured in the same manner as in Example 6 (Fig. 3), the emulsion adhesive solution having a viscosity of 150 Pa·s was applied in a thickness of 40 $\mu$m. As a result, leaking of the adhesive solution to the rear side of the substrate was not confirmed and coating by direct application method was possible. The hardness of loop of the pressure-sensitive adhesive sheet at this time was 26 mN.

EXAMPLE 9

After applying 40 g/m$^2$ of the filling agent in the solution concentration of 45% on a substrate including a polyethylene terephthalate nonwoven fabric having a bulk density of 0.30 g/cm$^3$ and manufactured by a melt blowing method having laminated on one side thereof a polyethylene terephthalate nonwoven fabric having a bulk density of 0.16 g/cm$^3$ manufactured by a spunbond method (Fig. 4), the emulsion adhesive solution having a viscosity of 50 Pa·s was applied in a thickness of 40 $\mu$m. As a result, leaking of the adhesive solution to the rear side of the substrate was not confirmed and coating by direct application method was possible. The hardness of loop of the pressure-sensitive adhesive sheet at this time was 15 mN.

COMPARATIVE EXAMPLE 9

After applying 20 g/m$^2$ of the filling agent in the solution concentration of 45% on the substrate manufactured in the same manner as in Example 6 (Fig. 3), the emulsion adhesive solution having a viscosity of 30 Pa·s was coated in a thickness of 40 μm. As a result, leaking of the adhesive solution to the rear side of the substrate was confirmed and coating by direct application method was impossible. The hardness of loop could not be measured, since the pressure-sensitive adhesive sheet could not be obtained.

COMPARATIVE EXAMPLE 10

After applying 20 g/m² of the filling agent in the solution concentration of 45% on the substrate manufactured in the same manner as in Example 6 (Fig. 3), the emulsion adhesive solution having a viscosity of 180 Pa·s was coated in a thickness of 40 μm. As a result, coating by direct application method was difficult, since the viscosity of the adhesive solution was high, although leaking of the adhesive solution to the rear side of the substrate was not confirmed. The hardness of loop of the pressure-sensitive adhesive sheet at this time was 30 mN.

The results of evaluations of the pressure-sensitive adhesive sheets obtained in Examples 6 to 9 and Comparative Examples 9 and 10 are shown in Table 4 below.

TABLE 4

| Example No. | Application Amount of Filling Agent (g/m²) | Coefficient of Viscosity of Adhesive (Pa's) | Leaking to Rear Side of Substrate | Coating Possibility of Adhesive | Hardness of Loop(mN) |
|---|---|---|---|---|---|
| Example 6 | 15 | 70 | No | Possible | 26 |
| Example 7 | 20 | 70 | No | Possible | 30 |
| Examples 8 | 15 | 150 | No | Possible | 26 |
| Example 9 | 40 | 50 | No | Possible | 15 |
| Comparative Example 9 | 20 | 30 | Yes | Possible | - |
| Comparative Example 10 | 20 | 180 | No | Impossible | 30 |

As described in detail above, the pressure-sensitive adhesive tapes or sheets for medical use in the invention, and the pressure-sensitive adhesive tapes or sheets for medical use obtained by the process not part of the invention are manufactured by a direct application method of an adhesive solution, so that the pressure-sensitive adhesive tapes or sheets are gentle to the environment, reduced in manufacturing costs and economical. Further, the pressure-sensitive adhesive tapes or sheets for medical use are hardly stuffy, and the surface of the substrate opposite to the pressure-sensitive adhesive layer has a good touch. Accordingly, the pressure-sensitive adhesive tapes or sheets for medical use can be used as pressure-sensitive adhesive tapes or sheets for medical use gentle to the skin. Specifically, the pressure-sensitive adhesive tapes or sheets in the invention can be used as a first aid sticking plaster, a wrapping type sticking plaster, a large sticking plaster with a pad, and a dressing member.

While the invention has been described in detail and with reference to specific embodiments thereof, it will be apparent to one skilled in the art that various changes and modifications can be made therein without departing from the scope thereof.

This application is based on Japanese patent application No. 2006-107114 filed April 10, 2006, Japanese patent application No. 2006-170163 filed June 20, 2006, and Japanese patent application No. 2006-220499 filed August 11, 2006, the entire contents thereof being hereby incorporated by reference.

Further, all references cited herein are incorporated in their entireties.

Claims

1. A pressure-sensitive adhesive tape or sheet, which comprises:

a support (1); and
a pressure-sensitive adhesive (2) layer disposed on one side of the support (1),
wherein the support comprises a first substrate (3) and a second substrate (4) disposed on at least one side of the first substrate (3), and
said first substrate (3) includes a thermoplastic melt blow nonwoven fabric having a thickness of from 5 μm to

50 µm,
**characterized in that**
said support (1) is treated with a filling agent chosen from the group of vinyl acetate series resins, acrylic series resins, styrene-butadiene rubber resins, and rosin series resins,
the amount of the filling agent is from 3 to 60 g/m$^2$,
said second substrate (4) includes a spunbond nonwoven fabric having a bulk density of from 0.05 to 0.3 g/cm$^3$, and
the second substrate (4) is arranged as the outermost layer (5) on the support side.

2. The pressure-sensitive adhesive tape or sheet according to claim 1, wherein the first substrate (3) and second substrate (4) are independently constituted of polyolefin, polyester, or polyamide.

3. The pressure-sensitive adhesive tape or sheet according to claim 1, wherein the filling agent is in a form of emulsion.

4. The pressure-sensitive adhesive tape or sheet according to claim 1, wherein the surface of the outermost layer (5) on the support side is subjected to a release treatment.

5. The pressure-sensitive adhesive tape or sheet according to claim 1, which is wound in a roll form.

**Patentansprüche**

1. Druckempfindliches Haftband oder druckempfindliche Haftfolie, das/die umfasst:

   einen Träger (1) und
   eine druckempfindliche Haftschicht (2), die auf einer Seite des Trägers (1) angeordnet ist,
   wobei der Träger ein erstes Substrat (3) und ein zweites Substrat (4) umfasst, das auf wenigstens einer Seite des ersten Substrates (3) angeordnet ist, und
   das erste Substrat (3) einen thermoplastischen schmelzgeblasenen Vliesstoff umfasst, der eine Dicke von 5 µm bis 50 µm hat,
   **dadurch gekennzeichnet, dass**
   der Träger (1) mit einem Füllmittel behandelt ist, das aus der Gruppe gewählt ist, die aus Harzen der Vinylacetatreihe, Harzen der Acrylreihe, Styren-Butadien-Kautschukharzen und Harzen der Kolophoniumreihe besteht,
   die Menge des Füllmittels im Bereich zwischen 3 und 60 g/m$^2$ liegt,
   das zweite Substrat (4) einen Spinnvliesstoff umfasst, der eine Rohdichte von 0,05 bis 0,3 g/cm$^3$ hat, und
   das zweite Substrat (4) als die äußerste Schicht (5) auf der Trägerseite angeordnet ist.

2. Druckempfindliches Haftband oder druckempfindliche Haftfolie nach Anspruch 1, bei dem/der das erste Substrat (3) und das zweite Substrat (4) unabhängig aus Polyolefin, Polyester oder Polyamid bestehen.

3. Druckempfindliches Haftband oder druckempfindliche Haftfolie nach Anspruch 1, bei dem/der das Füllmittel in Form einer Emulsion vorliegt.

4. Druckempfindliches Haftband oder druckempfindliche Haftfolie nach Anspruch 1, bei dem/der die Oberfläche der äußersten Schicht (5) auf der Trägerseite einer Ablösebehandlung unterzogen ist.

5. Druckempfindliches Haftband oder druckempfindliche Haftfolie nach Anspruch 1, das/die in Rollenform gewickelt ist.

**Revendications**

1. Bande ou feuille adhésive sensible à la pression, laquelle comprend :

   un support (1) ; et
   une couche adhésive sensible à la pression (2) disposée sur un côté du support (1),
   dans laquelle le support comprend un premier substrat (3) et un second substrat (4) disposés sur au moins un côté du premier substrat (3), et

ledit premier substrat (3) comprend un textile non tissé soufflé à l'état de fusion thermoplastique présentant une épaisseur de 5 μm à 50 μm,

**caractérisée en ce que**

ledit support (1) est traité avec un agent de remplissage choisi dans le groupe de résines des séries acétate de vinyle, de résines des séries acryliques, de résines de caoutchouc de styrène-butadiène, et de résines des séries colophane,

la quantité de l'agent de remplissage est de 3 à 60 g/m²,

ledit second substrat (4) comprend un textile non tissé filé lié présentant une masse volumique apparente de 0,05 à 0,3 g/cm³, et

le second substrat (4) est disposé comme la couche la plus externe (5) sur le côté de support.

2.  Bande ou feuille adhésive sensible à la pression selon la revendication 1, dans laquelle le premier substrat (3) et le second substrat (4) sont indépendamment constitués de polyoléfine, de polyester, ou de polyamide.

3.  Bande ou feuille adhésive sensible à la pression selon la revendication 1, dans laquelle l'agent de remplissage est dans une forme d'émulsion.

4.  Bande ou feuille adhésive sensible à la pression selon la revendication 1, dans laquelle la surface de la couche la plus externe (5) sur le côté de support est soumise à un traitement de détachement.

5.  Bande ou feuille adhésive sensible à la pression selon la revendication 1, laquelle est enroulée dans la forme de rouleau.

## FIG. 1

## FIG. 2

## FIG. 3

## FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3556082 B
- JP 10067652 A
- US 20030040691 A
- US 20030040691 A1

- JP 2006107114 A
- JP 2006170163 A
- JP 2006220499 A